# EUROPEAN PATENT APPLICATION

(11) **EP 4 458 191 A1**
(43) Date of publication of application: **06.11.2024**
(21) Application number: 21969994.9
(22) Date of filing: 28.12.2021
(51) Int. Cl.: A24F 40/53

(54) **AEROSOL GENERATION DEVICE, METHOD, AND PROGRAM**

(71) Applicant: Japan Tobacco Inc., Tokyo 105-6927 (JP)
(72) Inventor: FUJIKI, Takashi, Tokyo 130-8603 (JP); YOSHIDA, Ryo, Tokyo 130-8603 (JP)
(74) Representative: Hoffmann Eitle
(86) International application number: PCT/JP2021/048838
(87) International publication number: WO 2023/127113

(57) **Abstract**

When it is detected whether a smoking article is inserted into a device on the basis of a change in capacitance, electric power is consumed for capacitance detection. It is desirable to minimize the power consumption required for capacitance detection. An aerosol generation device for generating aerosol, comprising: a holding part for holding an aerosol forming base body comprising an aerosol source; a heater for heating the aerosol source; a detector for performing detection of a value of a capacitance sensor; and a controller which makes, by using the detected value of the capacitance sensor and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed; wherein the controller changes, based on a state of heating control relating to the heater, a sampling period used for detecting the value of the capacitance sensor.

## Description

### TECHNICAL FIELD

The present invention relates to an aerosol generation device, a method, and a program used for generating aerosol.

### BACKGROUND ART

In the field of a smoking device into which a smoking article is inserted to be heated for volatilizing components included in the smoking article, there is a technique to detect change in capacitance, as a technique for detecting whether a smoking article is being inserted in a smoking device (for example, Patent Literature 1).

### CITATION LIST

### PATENT LITERATURE

PTL 1: Japanese Patent Application Public Disclosure No. 2017-510270

### SUMMARY OF INVENTION

### TECHNICAL PROBLEM

In the case that detection as to whether or not a smoking article has been inserted in a device, that is based on change in capacitance, is performed, electric power is consumed for detecting the capacitance. It is desired to reduce the electric power required for capacitance detection as much as possible.

The present invention has been achieved in view of the above matters.

### SOLUTION TO PROBLEM

A mode of the present invention, for solving the above problems, comprises an aerosol generation device for generating aerosol which comprises: a holding part for holding an aerosol forming base body comprising an aerosol source; a heater for heating the aerosol source; a detector for performing detection of a value of a capacitance sensor; and a controller which makes, by using the detected value of the capacitance sensor and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed; wherein the controller changes, based on a state of heating control relating to the heater, a sampling period used for detecting the value of the capacitance sensor.

Further, a different mode of the present invention comprises the above aerosol generation device, wherein the controller changes the sampling period used for detecting the value of the capacitance sensor, depending on whether or not the heater is heating the aerosol forming base body.

Further, a different mode of the present invention comprises the above aerosol generation device, wherein the detector uses a sampling capacitor.

Further, a different mode of the present invention comprises the above aerosol generation device, wherein the sampling period during the time when the heater is heating the aerosol forming base body is longer than the sampling period during the time when heating is being stopped.

Further, a different mode of the present invention comprises the above aerosol generation device, wherein the sampling period during the time when the heater is heating the aerosol forming base body is shorter than the sampling period during the time when heating is being stopped.

Further, a different mode of the present invention comprises the above aerosol generation device, wherein the controller calculates a moving average value of the detected plural values of the capacitance sensor, and makes, by using the calculated moving average value and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

Further, a different mode of the present invention comprises the above aerosol generation device, wherein the threshold value is a threshold value relating to difference between the two moving average values, and the controller makes, by comparing the difference between the two moving average values with the threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

Further, a different mode of the present invention comprises the above aerosol generation device, wherein the controller is able to perform operation in a first mode and a second mode in which consumption of electric power is different from that in the first mode; the consumption of electric power in the second mode is smaller than that in the first mode; and, after heating of the aerosol forming base body by the heater is stopped, the mode of the aerosol generation device is changed to the second mode if a predetermined condition is satisfied, and the state in the second mode is canceled at predetermined time intervals to allow the detector to perform detection.

Further, a different mode of the present invention comprises the above aerosol generation device, wherein the number of the detectors is at least two.

Further, a different mode of the present invention comprises the above aerosol generation device, wherein the controller makes at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using each of the values of the capacitance sensors, that are detected in the at least two detectors, and the threshold value.

Further, a different mode of the present invention comprises a method performed by an aerosol generation device, which comprises a holding part for holding an aerosol forming base body comprising an aerosol source and a heater for heating the aerosol source, wherein the method comprises steps for: performing detection of a value of a capacitance sensor; and making at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using the detected value of the capacitance sensor and a predetermined threshold value; wherein the step for making the judgment changes, based on a state of heating control relating to the heater, a sampling period used for detecting the value of the capacitance sensor.

Further, a different mode of the present invention comprises a program which makes an aerosol generation device perform the above method.

### BRIEF DESCRIPTION OF DRAWINGS

Fig. 1 is a figure which schematically shows a construction example of an aerosol generation device according to an embodiment of the present invention.
Fig. 2 is a figure which shows a construction example in the case that an aerosol generation device according to an embodiment of the present invention constitutes a holder of a PCC.
Fig. 3 is a figure which shows an example of a construction of a capacitance sensor which may be used in an aerosol generation device according to an embodiment of the present invention.
Fig. 4 is a figure which is used for explaining an overview with respect to change in a count value (cnt) due to unevenness in quality of circuit components and effect relating to disturbance such as noise and so on.
Fig. 5 is a figure which is used for explaining calculation of a moving average of plural count values (ent).
Fig. 6 is a figure which is used for explaining an overview with respect to change in a count value (cnt) due to temperature drift in a sampling capacitor Cs.
Fig. 7 is a figure which is used for explaining difference between two count values (ent).
Fig. 8 is a figure which shows an example of a construction in which an aerosol generation device comprises plural detectors.
Fig. 9 is a figure which shows an example of positional relationship between a ground electrode and a sensor electrode of one sensor and a ground electrode and a sensor electrode of the other sensor.
Fig. 10 is a figure which shows an example of a sensing pattern for detecting capacitance.
Fig. 11 is a figure which shows an example of a construction in which an aerosol generation device comprises a detector arranged in a position close to a bottom part of a holding part.
Fig. 12 is a figure which shows an example of a case that a foreign substance in the form of a liquid has entered a holding part.
Fig. 13 is a figure which shows an example of a case that a foreign substance has entered a holding part.
Fig. 14 is a figure which shows an example of arrangement of a detector and a heater in an aerosol generation device according to an embodiment of the present invention.
Fig. 15 is a figure which shows a system construction example in the case that a notification is outputted to a user.
Fig. 16 is a figure which shows an example of a flow of a process performed in an aerosol generation device according to an embodiment of the present invention.
Fig. 17 is a figure which shows an example of a process flow of a process for detecting insertion of a stick-type base material that is performed in an aerosol generation device according to an embodiment of the present invention.

### DESCRIPTION OF EMBODIMENTS

In the following description, embodiments of the present invention will be explained in detail with reference to the figures.

### (Construction of Aerosol Generation Device)

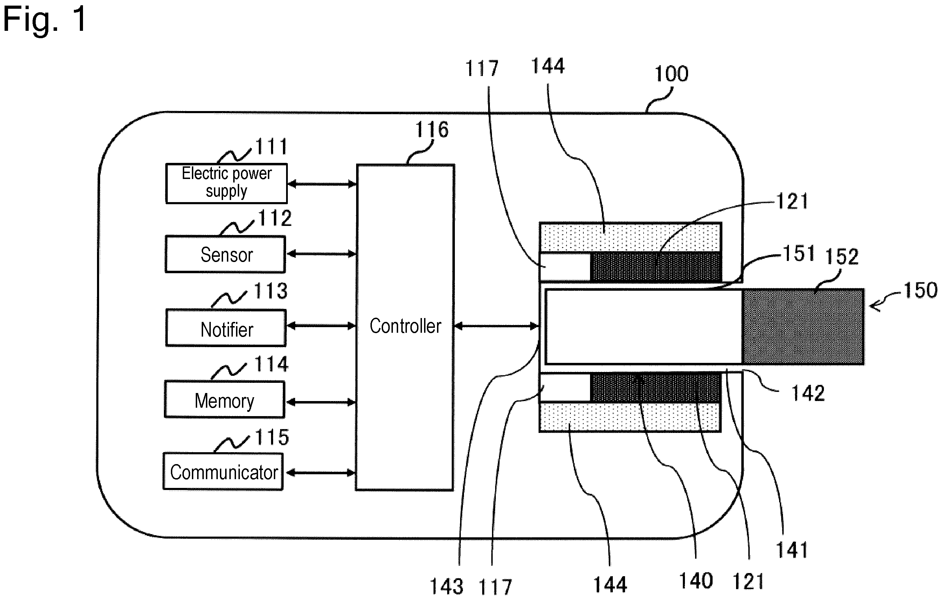

Fig. 1 is a figure which schematically shows a construction example of an aerosol generation device according to an embodiment of the present invention. As shown in Fig. 1, an aerosol generation device 100 according to the present construction example comprises an electric power supply 111, a sensor 112, a notifier 113, a memory 114, a communicator 115, a controller 116, a detector 117, a heater 121, a holding part 140, and a heat insulator 144. It should be reminded that, although the aerosol generation device 100 according to the present embodiment will be explained on the assumption that the aerosol generation device 100 is a heated tobacco product, the construction similar to that used in the aerosol generation device 100 can also be used in an electronic cigarette or the like. Further, although there are some kinds of heated tobacco products such as a low-temperature heated tobacco product, a high-temperature heated tobacco product, and so on which are categorized based on temperature of applied heat, it is possible to use, in any of the heated tobacco products, a construction similar to that used in the aerosol generation device 100 according to the present embodiment.

The electric power supply 111 stores electric power. The electric power supply 111 supplies, based on control performed by the controller 116, electric power to respective components in the aerosol generation device 100. The electric power supply 111 may comprise, for example, a rechargeable battery such as a lithium-ion secondary battery or the like. The electric power supply 111 may be charged by connecting it to an external electrical outlet via a USB (Universal Serial Bus) charging cable or the like (which is not shown in the figure). Further, it may be possible adopt a construction that the electric power supply 111 may be connected to a separate charger via a charging terminal which is not shown in the figure, and may receive electric power to be charged from the separate charger.

The sensor 112 obtains various kinds of information relating to the aerosol generation device 100. The sensor 112 may comprise a pressure sensor such as a microphone condenser or the like, a flow rate sensor, a temperature sensor, or the like. Further, the sensor 112 may comprise an input device such as a button, a switch, or the like which receives information input from a user. Further, the sensor may comprise a sensor constructed to detect the motion of a flavor inhaler or the like.

The notifier 113 notifies a user of information. The notifier 113 in the present embodiment may comprise a display device for displaying a message. The notifier 113 may comprise, for example, a voice outputting device such as a speaker or the like, a light emitting device or a light emitting element for emitting light, a display device for displaying images, a sound outputting device or an acoustic element for outputting sound, a vibration device including a vibrator for outputting vibration, or the like.

The memory 114 stores various kinds of information for operation of the aerosol generation device 100. The memory 114 comprises, for example, a nonvolatile storage medium such as a flash memory or the like. The memory 114 may comprise a volatile memory for providing a working area for control performed by the controller 116.

The communicator 115 may comprise a communication interface (including a communication module) which conforms to a predetermined LPWA wireless communication standard or a wireless communication standard defining regulations similar to those defined in the above standard. In this regard, Sigfox, LoRA-WAN, or the like may be adopted as the above communication standard. Further, the communicator 115 may comprise a communication interface which can perform communication conforming to any of other wired/wireless communication standards. For example, Wi-Fi (a registered trademark), Bluetooth (a registered trademark), or the like may be adopted as the above communication standard.

The detector 117 detects plural sensor values. In the present embodiment, the detector 117 is a capacitance sensor, for example. Further, in the case that the detector 117 is a capacitance sensor, the detector 117 may detect a value relating to parasitic capacitance as a sensor value, or, in the case that detector 117 comprises a sampling capacitor, may detect a value relating to the sampling capacitor. Further, the detector 117 may be an optical sensor such as an infrared proximity sensor or the like, a pressure sensor using a piezoelectric element, or the like. A sensor value detected by the detector 117 is used, by the controller 116 which will be explained later, for performing judgment as to whether a stick-type base material 150 has been inserted into an inner space 141 of the holding part 140 by a user of the aerosol generation device 100 or the like. In this regard, although the detector 117 is arranged in a position close to the bottom part 143 of the holding part 140 in Fig. 1, such arrangement is a mere arrangement example. The detector 117 may be arranged in a position other than the above position. Further, the number of detectors 117 installed in the aerosol generation device 100 may be one, or may be more than one. Matters relating to arrangement of the detector 117 will be explained later. In this regard, the detector 117 may be constructed as a part of the sensor 112.

The controller 116 functions as an arithmetic processing device and a control device, and controls overall operation of the aerosol generation device 100 in accordance with various kinds of programs. For example, the controller 116 is realized by using an electronic circuit such as a CPU (Central Processing Unit), a microprocessor, or the like.

Further, the controller 116 calculates a moving average value of plural sensor values of a capacitance sensor or the like, that are detected at the detector 117, and, by using the calculated moving average value and a predetermined threshold value, makes at least one of a judgment as to whether inserting of the stick-type base material 150 into the holding part 140 has been completed, a judgment as to whether or not the stick-type base material 150 has been inserted into the holding part, and a judgment as to whether extracting of the stick-type base material 150 from the holding part 140 has been completed. Further, judgment as to whether inserting of the stick-type base material 150 into the holding part 140 has been completed may be performed by using difference between plural moving averages and a predetermined threshold value relating to the difference. The moving average has been generally known as a method for making chronological data smooth. In the moving average, average values of fixed sections are obtained, respectively, and chronological change in the average value is shown. As a result, it becomes possible to further accurately determine the tendency of chronological change in a count value that will be explained later, for example. In this regard, a person skilled in the art will understand that any moving average method, such as a simple moving average method, a weighted moving average method, an exponential moving average method, or the like may be used in the present embodiment.

Further, the controller 116 may perform at least one of judgment as to whether inserting of the stick-type base material 150 into the holding part 140 has been completed, judgment as to whether or not the stick-type base material 150 has been inserted into the holding part, and judgment as to whether extracting of the stick-type base material 150 from the holding part 140 has been completed, by using a capacitance-sensor value detected by the detector 117 and a predetermined threshold value. That is, a construction for comparing the value of the capacitance sensor as it stands (a parasitic capacitance value, a sampling-capacitor value) with the threshold may be adopted. Further, the controller 116 may be constructed to be able to change, based on the state of heat control relating to the heater 121, a sampling period for detecting the capacitance-sensor value. For example, the controller 116 may be constructed to be able to change, according to whether the heater 121 is in the state that it is heating an aerosol forming base body (the stick-type base material 150), a sampling period for detecting the capacitance-sensor value.

The holding part 140 holds an aerosol forming base body. The holding part 140 has an inner space 141, and holds the stick-type base material 150 in such a manner that a part of the stick-type base material 150 is housed in the inner space 141. In this regard, the stick-type base material 150 in the present embodiment is an example of the aerosol forming base body, and is sometimes referred to as a "refill." In the present embodiment, the stick-type base material 150 is a slender-stick-shaped aerosol forming base body; however, it may have a different shape. The holding part 140 in the present embodiment has a long and narrow shape, that is similar the shape of the stick-type base material 150, as a whole for making it possible to house the stick-type base material 150 therein. The holding part 140 has an opening 142 which makes the inner space 141 communicate with the outside, and holds the stick-type base material 150 inserted via the opening 142 into the inner space 141. For example, the holding part 140 is a cylindrical body which comprises the opening 142 and a bottom part 143 which constitutes a bottom surface, and defines the columnar inner space 141. In the present embodiment, for example, the holding part 140 has a slender cylindrical shape extending in a direction of insertion of the stick-type base material 150. More specifically, in the present embodiment, the holding part 140 has a cylindrical shape, wherein the length of the cylindrical shape (the height) (the distance between the opening 142 and the bottom part 143) is longer than the width of the opening 142 (the opening 142 has an approximately circular shape in the present embodiment, and "the width of the opening 142" is a diameter, for example). The holding part 140 also has a function to define a flow path for air supplied to the stick-type base material 150. An air inflow hole, which is an air inlet of the flow path, is arranged in a position in the bottom part 143, for example. On the other hand, an air outflow hole, which is an air outlet of the flow path, is the opening 142.

The stick-type base material 150 comprises a base-material part 151 and a suction opening part 152. The base-material part 151 comprises an aerosol source. The aerosol source may be solid or liquid; and the aerosol source is atomized as a result that it is heated, and aerosol is generated accordingly. The aerosol source may be that which originates from tobacco, such as shredded tobacco, a product which is made by processing tobacco raw material to have a granular form, a sheet form, or a powder form, for example. Further, the aerosol source may be that which does not originate from tobacco, such as that made by use of a plant other than tobacco (for example, mint, a herb, and so on). For example, the aerosol source may comprise a flavor component such as menthol or the like. In the case that the aerosol generation device 100 is a medical inhaler, the aerosol source may comprise a medicine that is to be inhaled by a patient. In the state that the stick-type base material 150 is being held by the holding part 140, at least a part of the base-material part 151 is housed in the inside of the inner space 141, and at least a part of the suction opening part 152 protrudes from the opening 142. Thus, when the suction opening part 152, which protrudes from the opening 142, is held in a user's mouth and an inhalation action is performed by the user, air flows into the inner space 140 from the air inflow hole which is not shown in the figure, and the air, together with the aerosol generated in the base-material part 151, arrives at the inside of the user's mouth.

The heater 121 heats the aerosol source, which is included in the base-material part 151, to atomize the aerosol source to thereby generate aerosol. In the example shown in Fig. 1, the heater 121 is constructed to have a film shape, and is arranged to cover at least a part of the outer periphery of the holding part 140. Thus, when heat is generated by the heater 121, the base-material part 151 of the stick-type base material 150 is heated from at least a part of the outer periphery, and aerosol is generated thereby. The heater 121 generates heat when electric power is supplied thereto from the electric power supply 111. Further, in the present embodiment, the controller 116 may be constructed to be able to control the heater 121 to start heating, when it is judged that the stick-type base material 150 has been inserted into the inner space 141 of the holding part 140. Further, the controller 116 may be constructed to be able to control the heater 121 to stop heating, when it is judged that the stick-type base material 150 has been extracted from the inner space 141 of the holding part 140.

The heat insulator 144 prevents heat transfer from the heater 121 to other components. For example, the heat insulator 144 comprises vacuum insulation material, aerogel insulation material, or the like.

In the above description, construction examples of the aerosol generation device 100 have been explained. Although it is needless to state, the construction of the aerosol generation device 100 is not limited to any of those explained above, and it may have any of various constructions shown below as examples.

For example, the heater 121 may be constructed to have a blade shape, and arranged to protrude from the bottom part 143 to the inner space 141 of the holding part 140. In such a case, the blade-shape heater 121 is inserted into the base-material part 151 of the stick-type base material 150, and the base-material part 151 of the stick-type base material 150 is heated from the inside thereof. In a different example, the heater 121 may be arranged to cover the bottom part 143 of the holding part 140. Further, the heater 121 may be constructed as that comprising a combination of two or more of a first heater covering the outer periphery of the holding part 140, a second heater having a blade shape, and a third heater covering the bottom part 143 of the holding part 140.

In a different example, the holding part 140 may comprise an opening/closing mechanism, such as a hinge or the like, for opening/closing a part of an outer shell which forms the inner space 141. The holding part 140 may hold the stick-type base material 150 inserted into the inner space 141, by opening/closing the outer shell. In such a case, the heater 121 may be arranged in the position where the stick-type base material 150 is to be held in the holding part 140, and may heat the stick-type base material 150 while it is being pressed thereby.

Further, the means for atomizing the aerosol source is not limited to heating by the heater 121. For example, the means for atomizing the aerosol source may be induction heating.

The aerosol generation device 100 illustrated in Fig. 1 itself may constitutes a heated tobacco product, or it may constitute a holder of a PCC (Portable Charger Case). That is, the aerosol generation device 100 may be constructed in such a manner that the aerosol generation device 100 is electrically connected, via a charging terminal or the like, to a separate charger which is not shown in the figure, and a quantity of electric power, that allows several number of times of smoking, is charged from the charger to a rechargeable battery (electric power supply 111).

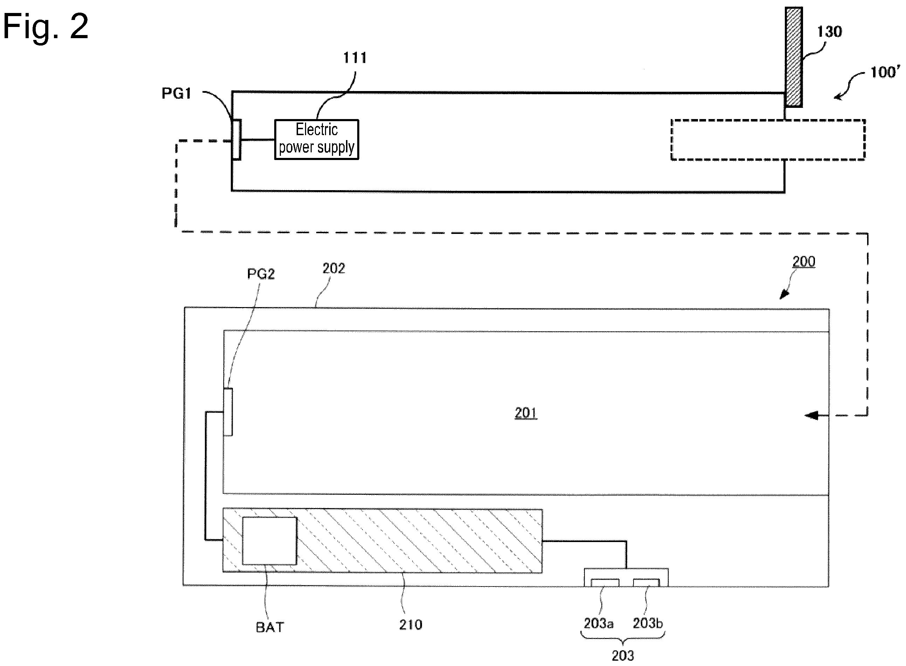

Fig. 2 is a figure which shows a construction example of a PCC system in the case that the aerosol generation device 100 constitutes a holder of a PCC. The PCC system illustrated in Fig. 2 comprises a holder 100' and charger (battery charger) 200. Further, the holder 100' further comprises, in addition to the construction of the aerosol generation device 100 shown in Fig. 1, a charging terminal PG1 for connection to the charger 200 for allowing charging through it. The charging terminal PG1 is connected to the electric power supply 111, and the charging terminal PG1 sends electric power supplied from the charger 200 to the electric power supply 111. In Fig. 2, although the construction of the holder 100' is depicted in a simplified manner, the construction of the holder 100', except for the charging terminal PG1, is similar to that shown in Fig. 1.

The charger 200 may be constructed to supply electric power to the holder 100' for charging the electric power supply 111 in the holder 100'. For example, the charger 200 illustrated in Fig. 2 may be a portable pocket charger, and may have a size that allows the charger 200 to be carried in a bag or a pocket of a garment of a user. The charger 200 may comprise, for example, a housing 202 having a container 201 (housing space) which can house the holder 100', a user interface 203, and an electric component 210 having an electric power supply BAT. The electric power supply BAT may be constructed by using a secondary battery such as a lithium-ion secondary battery, or an electric double layer capacitor such as a lithium-ion capacitor. The user interface 203 may comprise, for example, a display part 203a for providing a user with information (for example, a light emitting element such as an LED or the like, and/or an image displaying device such as an LCD or the like), and/or a manipulation part 203b for receiving manipulation performed by a user (for example, a switch such as a button switch or the like, and/or a touch display). The electric component 210 is installed in the housing 202.

The whole holder 100' or a part of the holder 100' including the charging terminal PG1 is inserted into the container 201 in the charger 200, as depicted by an arrow with a broken line in Fig. 2. Further, the charger 200 may comprise, in the container 201, a connector PG2 which is electrically connected to the charging terminal PG1 of the holder 100' when the whole holder 100' or a part of the holder 100' including the charging terminal PG1 is inserted into the container 201. In this regard, the charger 200 may comprise, for example, a terminal such as a USB terminal or the like (which is not shown in the figure) which is to be electrically connected to a domestic power source for charging the electric power supply BAT in the charger 200. Further, the charger 200 may comprise, in the housing 202 thereof, a lid member (which is not shown in the figure) constructed to be able to open/close the container 201, to cover the holder 100' housed in the container 201.

### (Capacitance Sensor)

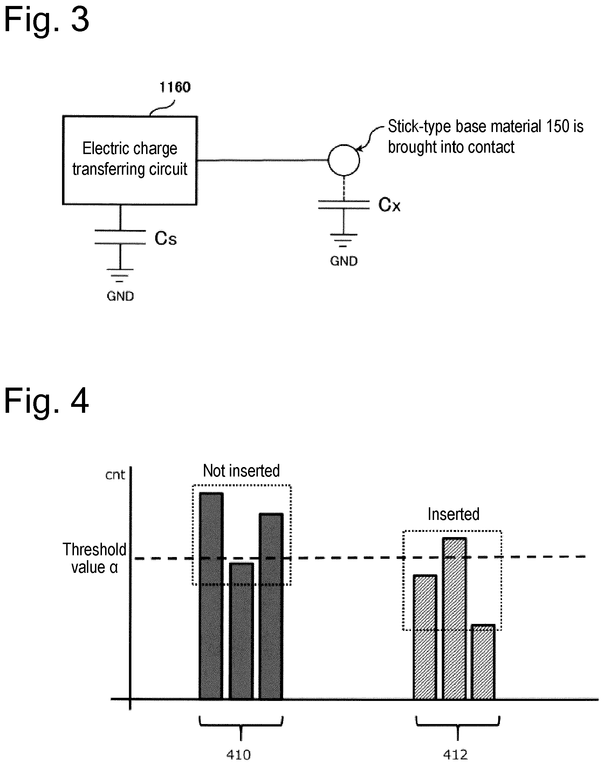

As explained above, the detector 114 may be a capacitance sensor. Fig. 3 is a figure which shows an example of a construction of a capacitance sensor which may be used in the aerosol generation device 100 according to the present embodiment. Controlling of the capacitance sensor is performed by the controller 116. The electric charge stored in a capacitor Cx (parasitic capacitance) increases if the stick-type base material 150 is inserted into the holding part 140. With respect to the capacitance sensor, action for transferring the electric charge in the capacitor Cx to a sampling capacitor Cs is repeated at predetermined intervals by controlling an electric charge transferring circuit 1160 by the controller 116, and the controller 116 counts the number of times of transfer actions performed. The controller 116 comprises a register which is not shown in the figure, and records the count value (i.e., the number of times of actions for transferring the electric charge in the capacitor Cx to the sampling capacitor Cs) in the register, every time when the terminal voltage of the sampling capacitor Cs has reached a threshold value Vih. In this regard, the count value may be recorded in the memory 114. By performing control such as that explained above, the controller 116 can judge whether the stick-type base material 150 has been inserted in the holding part 140 and/or extracted from the holding part 140. Regarding the capacitor Cs and the capacitor Cx, they are constructed in such a manner that the capacitance of the capacitor Cs is larger than the capacitance of the capacitor Cx.

Further, the electric charge transferring circuit 1160 comprises a switch; and switching between charging of the capacitor Cx with electric charge and transferring of electric charge to the sampling capacitor Cs is realized by controlling the switch by the controller 116. The controller 116 performs control operation in such a manner that the switch is closed at predetermined intervals for connecting the capacitors Cx and Cs with each other, and the switch between the capacitors Cx and Cs is opened during the time when capacitor Cx is charged.

For example, in the case that the number of times of actions for transferring electric charge in the capacitor Cx to the sampling capacitor Cs, that were performed until when the terminal voltage of the sampling capacitor Cs has reached the threshold value Vih, in the state that the stick-type base material 150 is not being inserted into the holding part 140, is 8000, "count value (cnt) = 800" is recorded in a register included in the controller 116. The electric charge in the capacitor Cx increases when the stick-type base material 150 is brought into contact with the capacitance sensor (the detector 117), so that the count value at the time when the terminal voltage of the sampling capacitor Cs has reached the threshold value Vih decreases. That is, the terminal voltage of the sampling capacitor Cs reaches the threshold value Vih at the time when the count value has reached a count value smaller than a count value that is expected in the case that the stick-type base material 150 is not being inserted into the holding part 140. That is, based on difference between the count value obtained in the state that the stick-type base material 150 is not being inserted into the holding part 140 and the count value obtained in the state that the stick-type base material 150 is being inserted into the holding part 140, the controller 116 can detect whether or not the stick-type base material 150 is being inserted into the holding part 140, or the like.

In this regard, the length of time that is spent until the terminal voltage of the sampling capacitor Cs reaches the threshold value Vih is that approximately in the range of several milliseconds (ms) to several tens of milliseconds. Thus, the above is regarded as a one cycle, and the cycle is repeated at certain specific intervals (hereinafter, a "sampling period") during the time when the capacitance sensor is in an active state.

Further, in a different embodiment, the controller 116 may be constructed to be able to change the sampling period for detecting the capacitance-sensor value (and/or the number of times of sampling in the case that a moving average is to be calculated as explained below) according to the state of control of operation of the aerosol generation device 100.

The controller 116 may operate the aerosol generation device in one of plural modes in which the quantities of the maximum electric power consumption, that are peak values of quantities of consumption of electric power stored in the electric power supply 111, are different from one another. For example, the controller 116 changes the operation mode, by changing the number of hardware components used for operating the aerosol generation device 100, changing the circuit that is to be operated, or the like. Examples of the operation modes are an activation state during that the controller 116 makes all hardware components operate as necessary, a sleep state during that the quantity of the maximum electric power consumption is reduced compared with that in the activation state, and so on. For example, in the sleep state, the controller 116 stops operation of hardware components except for the controller 116, and invalidates functions in the controller 116 other than the function for detecting manipulation performed on an input device which accepts information input from a user.

In the sleep state during that the quantity of the maximum electric power consumption is reduced compared with that in the activation state, the aerosol generation device 100 changes its state to the activation state in response to reception of user's manipulation input (button manipulation, slider manipulation or the like), detection of insertion of the stick-type base material 150 during a state that the sleep state is being cancelled temporarily, that will be explained later, or the like. The controller 116 may start heating control in response to detection of insertion of the stick-type base material 150 in the activation state. The controller 116 may enter an operation state, and, further, start heating control, based on detection of insertion of the stick-type base material 150 in the state that the sleep state is being cancelled temporarily, that will be explained later. The controller 116 stops heating of the aerosol forming base body (the stick-type base material 150) by the heater 121, for example, when a predetermined length of time has elapsed since a start of heating, when completion of a predetermined number of times of puffs is detected, when extraction of the stick-type base material 150 is detected, or the like. After stopping heating, the controller 116 may change, automatically after a predetermined length of time has elapsed or in response to reception of user's manipulation input (button manipulation, slider manipulation or the like), the state of the aerosol generation device 100 to the sleep state during that the quantity of the maximum electric power consumption is reduced compared with that in the activation state.

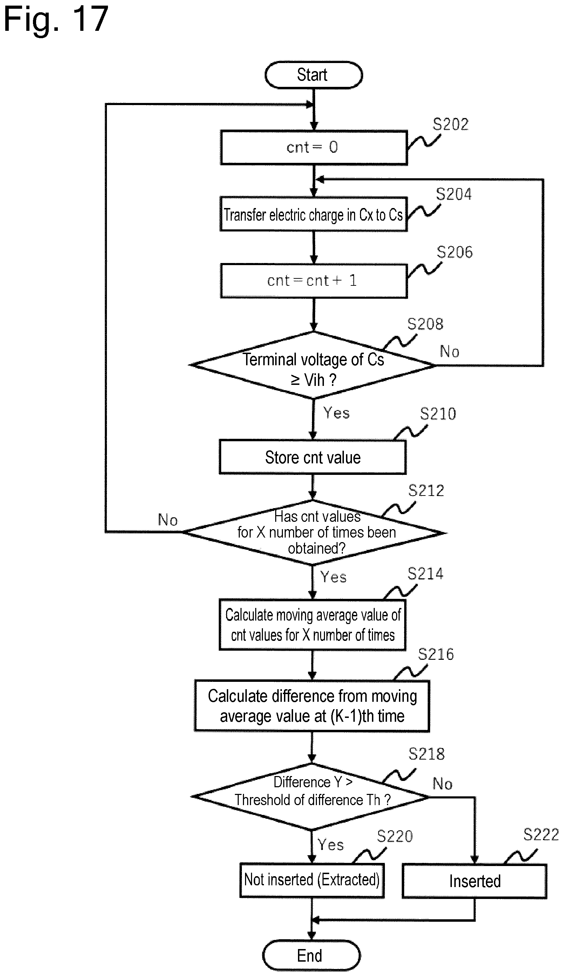

For example, the controller 116 may be realized by using a MCU (Micro Controller Unit), and may control operation of the heater 121 based on a heating profile stored in the memory 114. A heating profile is information that defines chronological change in target temperature that is the target value of temperature of the heater 121. The controller 116 obtains temperature information of the heater 121, calculates difference between the temperature indicated by the temperature information and the target temperature, and performs publicly-known feedback control such as PID control or the like, for example. Specifically, electric power from an electric power supply, that is made to have the form of a pulse formed by performing pulse width modulation (PWM) or pulse frequency modulation (PFM), is supplied to the heater 121; and the controller 116 makes electric power be supplied to the heater 121, and, while electric power is being supplied, calculates, based on the difference between the temperature information and the target temperature of the heater 121, a duty ratio of the electric pulse and adjusts it. The controller 116 may be constructed to change the sampling period for detecting the capacitance-sensor value, depending on whether or not the heater 121 is heating the aerosol forming base body (the stick-type base material 150). Further, in the case that the controller 116 is constructed to change the sampling period, if the controller 116 is constructed to calculate a moving average value of count values in relation to detection of insertion/extraction of the stick-type base material 150 as explained later, the controller 116 may increase/decrease the number of times of sampling (the value of the variable X in Fig. 17) performed for calculating the moving average, in addition to or in place of changing the sampling period.

The controller 116 performs heating control during heating as explained above, so that the load on the MCU and so on becomes high. Thus, in view of reduction of the load on the MCU and so on and saving of electric power, the controller 116 may make, during heating, the sampling period long compared with that in the time when heating is being stopped (the time before a start of heating and/or the time after a stop of heating). Further, the number of times of sampling, in the case that a moving average is to be calculated as explained later, may be reduced. By adopting the above construction, it becomes possible to precisely detect, in the state that heating is being stopped, insertion/extraction of the stick-type base material; and it becomes possible to detect, in the state that heating is being performed, insertion/extraction of the stick-type base material 150, while reducing the load on the MCU and so on and saving electric power.

Further, the controller 116 may be constructed to make the sampling period short while heating is being performed, compared with the sampling period in the time when heating is being stopped (the time before a start of heating and/or the time after a stop of heating). This is because there may be a case that accuracy of detection by the detector 117 is lowered due to change in capacitance due to temperature drift that occurs as a result of change in temperature as explained later, or due to other reasons, during the time when heating control is being performed. Thus, accuracy of detection by the detector 117 can be maintained by making the sampling period short. Further, it may be possible to increase the number of times of sampling, in the case that a moving average is to be calculated as explained later. By adopting the above construction, it becomes possible to precisely detect, in the state that heating is being performed, insertion/extraction of the stick-type base material 150; and it becomes possible to detect, in the state that heating is being stopped, insertion/extraction of the stick-type base material 150, while saving electric power.

After stopping heating of the aerosol forming base body (the stick-type base material 150) by the heater 121, the controller 116 may change, automatically after a predetermined length of time has elapsed or in response to reception of user's manipulation input (button manipulation, slider manipulation or the like), the state of the aerosol generation device 100 to the sleep state during that the quantity of the maximum electric power consumption is reduced compared with that in the activation state, and, thereafter, may cancel the sleep state at predetermined time intervals, and make the detector 117 perform detection operation. The state that the sleep state has been canceled may correspond to the activation state or a partial activation state. The partial activation state refers to a state that the sleep state of part of the circuit or hardware construction, which relates to detection of insertion/extraction of the stick-type base material 150 performed by the detector 117 and the controller 116, only is canceled, and the sleep state of part of the circuit or hardware construction, which does not relate to the insertion/extraction detection, is maintained. By performing the process for detecting insertion/extraction of the stick-type base material 150 by temporarily canceling the sleep state at predetermined timing even if the present stats is the sleep state, it becomes possible to perform, while saving electric power, a process for detecting insertion of the stick-type base material 150 at the time when a user attempts smoking, and performing heating immediately, and so on, for example.

On the other hand, the count value (cnt) detected by the capacitance sensor varies for the reasons listed below. That is, in addition to the case when the stick-type base material 150 is being inserted into the holding part 140, there may be cases that the count value (cnt) may vary for the following reasons:
(a) Unevenness in quality of circuit components
(b) Effect of disturbance such as noise and so on.

Fig. 4 is a figure which is used for explaining an overview with respect to change in a count value (cnt) due to (a) unevenness in quality of circuit components and (b) effect of disturbance such noise and so on. Fig. 4 depicts plural graphs 410 that represent count values (cnt) counted in the state that the stick-type base material 150 is not being inserted into the holding part 140, and plural graphs 412 that represent count values (cnt) counted in the state that the stick-type base material 150 is being inserted into the holding part 140, in an environment at a certain temperature. In this regard, it should be reminded that the graphs shown in Fig. 4 are those for showing an overview of change in the count value (cnt) due to the reasons shown in above items (a) and (b), and are not those precisely showing change in the count value. Further, each of the graphs shown in Fig. 4 shows the number of times that the terminal voltage of the sampling capacitor Cs reached the threshold value Vih (count value).

As shown in Fig. 4, due to unevenness in quality of circuit components, or effect of noise such as circuit noise, external noise, and so on, the count value (cnt) detected by the detector 117 may vary. For example, the graphs 410 represent plural count values obtained in the state that the stick-type base material 150 is not being inserted into the holding part 140, and the respective count values are uneven. Further, although the count value usually exceeds the threshold value α when the stick-type base material 150 is not being inserted into the holding part 140, a count value that does not exceed the threshold value α exists in the count values. Also, the graphs 412 represent count values obtained in the state that the stick-type base material 150 is being inserted into the holding part 140, and the respective count values are uneven. Further, although the count value does not usually exceed the threshold value α when the stick-type base material 150 is being inserted into the holding part 140, a count value that exceeds the threshold value α exists in the count values. If the count values become uneven due to the reasons shown in above items (a) and (b) as explained above, the controller 116 cannot make a correct judgment with respect to the state of insertion of the stick-type base material 150. In a portable device such as a smoking device or the like, it is necessary to downsize the detector 117 in the capacitance sensor, and, accordingly, the capacitance that can be detected becomes small; thus, especially, unevenness of the count values (cnt) has effect on detection with respect to the state of insertion of the stick-type base material 150.

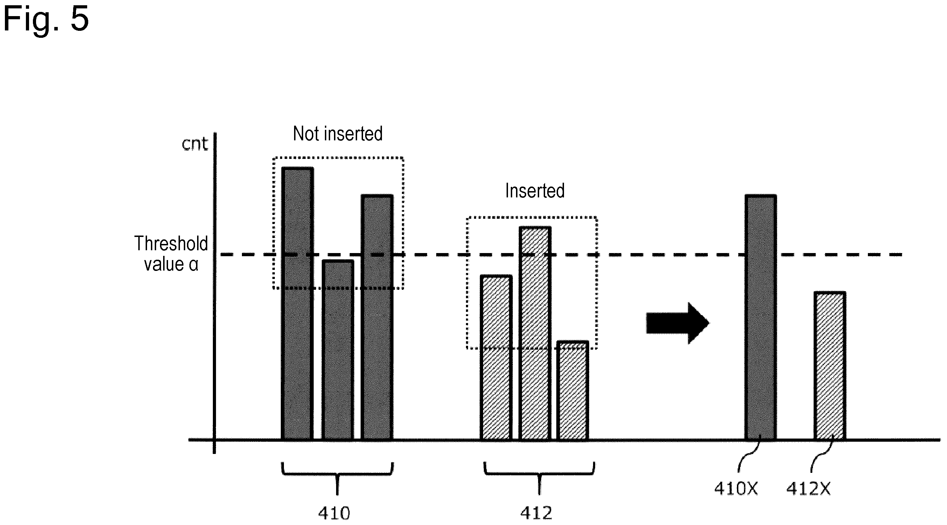

Fig. 5 is a figure which is used for explaining calculation of a moving average of plural count values. By taking a risk that the count values (cnt) may vary due to noise or the like explained in relation to Fig. 4 into consideration, the controller 116 can compensate for errors included in respective count values by calculating a moving average value (the graph 410x and the graph 420x) of count values obtained in a predetermined number of successive sampling processes. Thereafter, the controller 116 compares the calculate moving average with a threshold value that has been determined in advance in relation to the moving average, and, if the moving average is smaller than the threshold value, the controller 116 can judge that the stick-type base material 150 has been inserted into the holding part 140. In this regard, unevenness of values due to the reasons shown in above items (a) and (b) may also occur in terms of the capacitor Cx; thus, the controller 116 may be constructed to be able to calculate a moving average of detected values of the capacitor Cx in the case that the detector 117 also detects the value of the capacitor Cx.

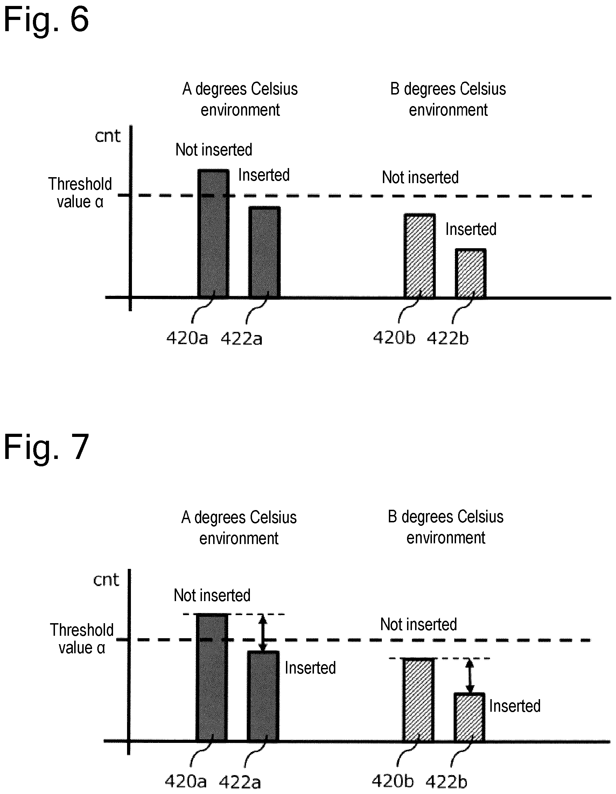

Further, the count value (cnt) may vary due to temperature drift in the sampling capacitor Cs. Fig. 6 is a figure which is used for explaining an overview with respect to change in the count value (cnt) due to temperature drift in the sampling capacitor Cs. Fig. 6 depicts a graph 420a that represents a count value (cnt) counted in the state that the stick-type base material 150 is being inserted into the holding part 140, and a graph 422a that represents a count value (cnt) counted in the state that the stick-type base material 150 is not being inserted into the holding part 140, in an environment at A degrees Celsius. Further, Fig. 6 depicts a graph 420b that represents a count value (cnt) counted in the state that the stick-type base material 150 is being inserted into the holding part 140, and a graph 422b that represents a count value (cnt) counted in the state that the stick-type base material 150 is not being inserted into the holding part 140, in an environment at B degrees Celsius that is different from A degrees Celsius. In this regard, it should be reminded that the respective graphs shown in Fig. 6 are those for showing an overview of change in the count value (cnt) due to temperature drift in the sampling capacitor Cs, and are not those for precisely showing change in the count value. Further, each of the graphs shown in Fig. 6 shows, for each sampling period, the number of times (cnt) that the terminal voltage of the sampling capacitor Cs reached the threshold value Vih.

As shown in Fig. 6, in the environment at A degrees Celsius, the graph 420a relating to the state that the stick-type base material 150 is not being inserted into the holding part 140 exceeded the threshold value α, and the graph 422a relating to the state that the stick-type base material 150 is not being inserted into the holding part 140 did not exceed the threshold value α. As explained above, based on whether or not the count value (cnt) has exceeded the threshold value, the controller 116 can judge whether or not the stick-type base material 150 has been inserted into the holding part 140. On the other hand, as a result of change in the capacitance of the sampling capacitor Cs due to temperature drift, there may be a case that both count values relating to the insertion state and the non-insertion state are decreased, and, thus, they do not reach the threshold value α, as shown by the graphs 420b and 422b. In such a case, even if the stick-type base material 150 is not being inserted into the holding part 140 (the graph 420b), the controller 116 may erroneously make a judgment indicating the state that the stick-type base material 150 has been inserted into the holding part 140.

For solving the above problems, instead of comparing an absolute value of the count value with the threshold value, the controller 116 may compare difference between two count values with a threshold value that has been determined in advance in relation to the difference. Further, the controller 116 may calculate a moving average value of plural count values explained in relation to Fig. 5, and compare the calculated moving average value with a threshold value that has been determined in advance in relation to the moving average value.

Fig. 7 is a figure which is used for explaining difference between two count values. In the case that temperature drift in the sampling capacitor Cs such as that explained in relation to Fig. 6 is to be occurred, the respective count values represented by the graph 420a and the graph 422a, on the whole, shift similarly when the environment temperature changes. Thus, by comparing difference between count values obtained before and after a count value obtained at each sampling period with a threshold value that has been determined in advance in relation to the difference, instead of comparing the absolute value of the count value obtained at each sampling period with the threshold value α, the controller 116 can compensate for temperature drift due to change in environment temperature. It is assumed that the threshold value that has been determined in relation to the difference is a value that is relatively large and allows to distinguish between the state that stick-type base material 150 is being inserted and the state that stick-type base material 150 is not being inserted. That is, in the case that a difference that does not exceed the threshold value is obtained, it can be judged that the last insertion state or the last non-insertion state has not been changed to the other state. Further, in the case that a difference that exceeds the threshold value is detected, it can be judged that the last insertion state has been changed to the non-insertion state, or the last non-insertion state has been changed to the insertion state. In this regard, temperature drift may similarly occur in the capacitor Cx; thus, the controller 116 may be constructed to be able to calculate a moving average of detected values of the capacitor Cx in the case that the detector 117 detects the value of the capacitor Cx.

Further, the aerosol generation device 100 according to the present embodiment may be constructed to be able to calculate a moving average value of count values or parasitic capacitance values obtained at predetermined number of sampling periods, further calculate difference between calculated moving averages, and compare the difference with a predetermined threshold value. By adopting the above construction, effect due to all of the reasons shown in above items (a)-(c) can be reduced.

### (Method for Arranging Sensor)

The aerosol generation device 100 according to the present embodiment may comprise plural detectors 117. Further, the controller 116 may make a judgment indicating the state that the stick-type base material 150 has been inserted into the holding part 140, when the moving average value of the capacitance-sensor values detected by each of the detectors 117 has exceeded the threshold value.

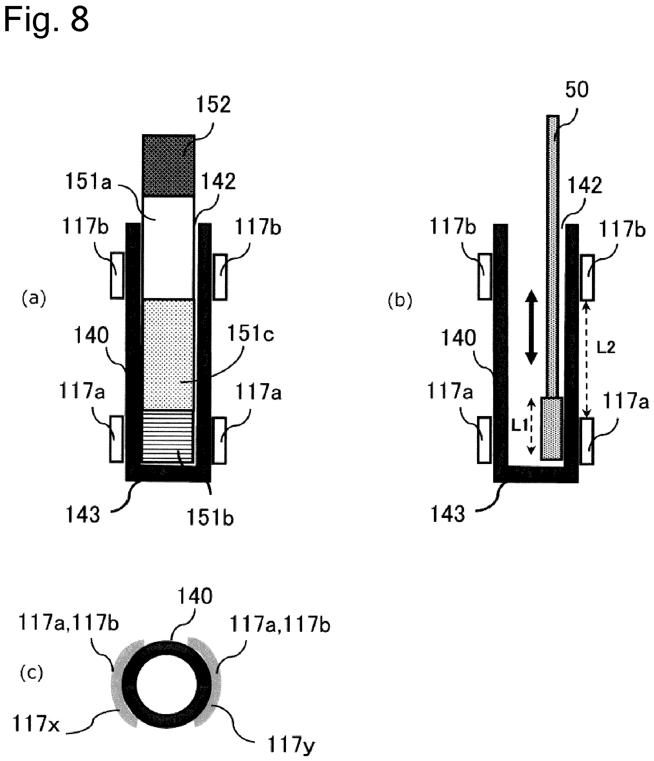

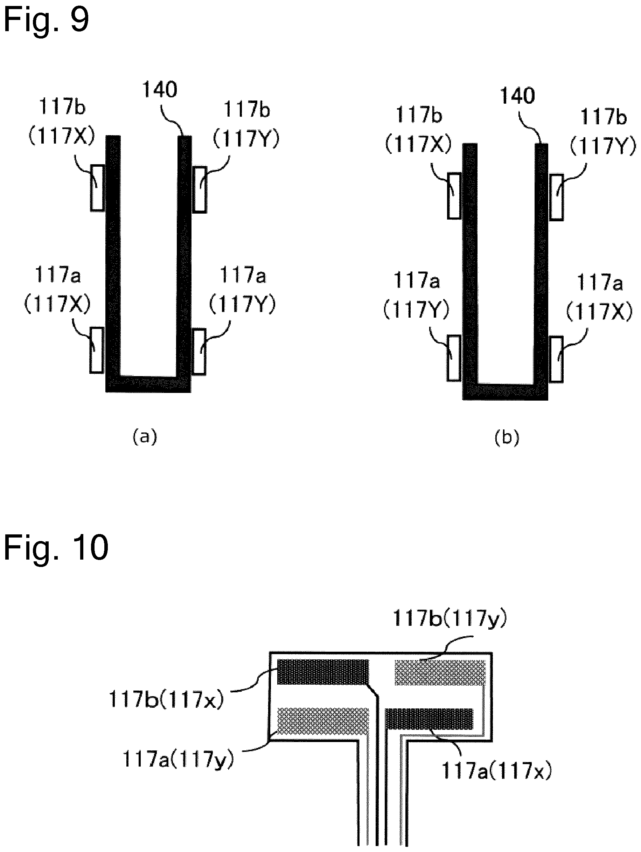

Fig. 8 is a figure which shows an example of a construction in which an aerosol generation device 100 comprises plural sensors (detectors 117), and shows the holding part 140 which is holding the stick-type base material 150 and positions of the detectors 117 in the aerosol generation device 100. The suction opening 152 of the stick-type base material 150 may comprise a filter; and the base-material part 151 may be constructed to include a paper tube 151a on the suction-opening side, a paper filter 151b at an end on a side opposite to the suction-opening side, and a material part 151c positioned between the paper tube 151a and the paper filter 151b and comprising a flavor source such as tobacco raw material, a flavoring agent, or the like, or medicine, or the like. The holding part 140 in the aerosol generation device 100 illustrated in Fig. 8 has a slender cylindrical shape extending in a direction of insertion of the stick-type base material 150; and (a) and (b) in Fig. 8 are cross-section views in the case that the holding part 140 in the aerosol generation device 100 is cut along a plane in which a central axis of the cylindrical shape in the longitudinal direction is included (Fig. 9 and Figs. 11-14 are also cross-section views similar to the above cross-section views). (a) in Fig. 8 shows a state that the stick-type base material 150 is being inserted into the holding part 140 in the aerosol generation device 100. (b) in Fig. 8 shows a state that the stick-type base material 150 is not being inserted into the holding part 140 in the aerosol generation device 100. (c) in Fig. 8 is a cross-section view in the case that the aerosol generation device 100 is cut along a plane that is substantially parallel with the bottom part 143 of the circular-shape holding part 140 in the aerosol generation device 100 and crosses sensors 117a and 117b. Each of the sensors 117a and 117b is a sensor which is a component of the detector 117 and is used for detecting capacitance of the capacitor Cx. Each of the sensors 117a and 117b may further be connected to the sampling capacitor Cs to constitute the detector 117.

The aerosol generation device 100 shown in each of (a), (b), and (c) in Fig. 8 comprises, for example, two sensors 117a and 117b. Further, the respective sensors 117a and 117b are arranged in positions, that are on a side surface of the cylindrical-shape aerosol generation device 100, in such a manner that they are separated from each other. The sensor 117a is arranged in a position that is close to the bottom part 143 of the holding part 140 and also roughly corresponds to a position of the paper filter 151b in the base-material part 151 in the stick-type base material 150 in the state that the stick-type base material 150 is being inserted into the holding part 140 ((a) in Fig. 8). Further, the sensor 117b is arranged in a position close to the opening 142 of the holding part 140.

With respect to the aerosol generation device 100, there may be a case that cinders of tobacco leaves, for example, remain on the bottom part 143 of the holding part 140 after heating of the stick-type base material 150 (after smoking performed by a user, or the like), and the inside of the holding part 140 is required to be cleaned by using a cleaning swab 50 or the like as shown in (b) in Fig. 8. In the above case, if the aerosol generation device 100 comprises the sensor 117a only, there may be possibility that the cleaning swab 50 may erroneously be detected as the stick-type base material 150. The aerosol generation device 100 according to the present example comprises, in addition to the sensor 117a, the sensor 117b arranged in a position close to the opening 142 of the holding part 140; thus, when the cleaning swab 50 is moved in the inside of the holding part 140, the probability that the both sensors 117a and 117b erroneously detect, at the same time, the cleaning swab 50 as the stick-type base material 150 becomes very low. In the case that the two sensors are arranged in positions distant from each other, especially, one position close to the bottom part 143 and the other position close to the opening 142 in the holding part 140 as explained above, respectively, that is, in the case that the two sensors are arranged in positions in both ends in the longitudinal direction in the inner space 141 of the holding part 140, respectively, possibility of occurrence of erroneous detection by the detector 117 and the controller 116 can be lowered.

It is preferable that a distance L2 between the sensor 117a and the sensor 117b be determined in the manner explained below. For example, a sensing pattern used for detecting capacitance is formed by using an FPC (Flexible Printed Circuit) or the like, and the sensor 117b is arranged in a position that is separated from the bottom part 143 of the holding part 140 or the sensor 117a by a distance L1 that is longer than the size of a conceivable foreign substance (the cleaning swab 50 in Fig. 8). That is, it is preferable to set the distances in such a manner that L1<L2.

Further, each of the sensors 117a and 117b may be constructed in such a manner that a single electrode faces an object of detection, or it may be constructed in such a that plural electrodes face one another via an object of detection. For example, as shown in (c) in Fig. 8, the sensors 117a and 117b are constructed by using two electrodes which face each other, and the two electrodes are arranged in such a manner that the side face of the holding part 140 is to be positioned between them. Each of the facing electrodes may have a flat surface or a curved surface. In the case that the above construction is adopted, the detector 117 may regard the two facing electrodes as those constituting the capacitor Cx, and detect, as a capacitance-sensor value, the capacitance between the two electrodes, or may detect, as a capacitance-sensor value, a count value detected by using the sampling capacitor Cs connected to the capacitor Cx. In this regard, the number of sensors may be three or more.

In the two electrodes which are components of the sensors 117a and 117b, one electrode is a ground electrode 117x connected to the ground, and the other electrode facing the one electrode is a sensor electrode 117y for reading electric charge stored in the capacitor Cx. Fig. 9 is a figure which shows an example of positional relationship between the ground electrode 117x and the sensor electrode 117y of the sensor 117a and the ground electrode 117x and the sensor electrode 117y of the sensor 117b. As shown in (a) and (b) in Fig. 9, the ground electrode 117x and the sensor electrode 117y of the sensor 117a are installed in such a manner that the electrodes face each other in the direction perpendicular to the longitudinal direction of the holding part 140 and the holding part 140 is put between the electrodes. Further, the distance from the opening 142, or the bottom part 143, to the ground electrode 117x of the sensor 117a and the distance from the opening 142, or the bottom part 143, to the sensor electrode 117y of the sensor 117a are substantially the same with each other. Similar to the above, the ground electrode 117x and the sensor electrode 117y of the sensor 117b are installed in such a manner that the electrodes face each other in the direction perpendicular to the longitudinal direction of the holding part 140 and the holding part 140 is put between the electrodes. Further, the distance from the opening 142, or the bottom part 143, to the ground electrode 117x of the sensor 117b and the distance from the opening 142, or the bottom part 143, to the sensor electrode 117y of the sensor 117b are substantially the same with each other.

Further, as shown in (a) in Fig. 9, the ground electrode 117x of the sensor 117a and the ground electrode 117x of the sensor 117b, and the sensor electrode 117y of the sensor 117a and the sensor electrode 117y of the sensor 117b may be arranged in such a manner that the positions of the ground electrodes substantially overlap with each other and the positions of the sensor electrodes substantially overlap with each other, in the longitudinal direction of the slender-cylindrical-shape holding part 140 (i.e., when they are viewed in the direction from the opening 143 to the bottom part 143). In a different construction, the ground electrode 117x of the sensor 117a and the sensor electrode 117y of the sensor 117b may be arranged in such a manner that the positions of the ground electrode and the sensor electrode substantially overlap with each other, and the sensor electrode 117y of the sensor 117a and the ground electrode 117x of the sensor 117b may be arranged in such a manner that the positions of the sensor electrode and the ground electrode substantially overlap with each other, in the longitudinal direction of the slender-cylindrical-shape holding part 140 (i.e., when they are viewed in the direction from the opening 143 to the bottom part 143). In this regard, the expression "substantially overlap" means that exact overlapping of positions is not necessarily required, that is, a minor position shift that still allows the electrodes to function as a capacitor is allowable.

Fig. 10 is a figure which shows an example of a sensing pattern for detecting capacitance. The sensing pattern used for detecting capacitance is formed by using an FPC or the like, and wound around the holding part 140 in such a manner that the ground electrode 117x and the sensor electrode 117y of the sensor 117b face each other via the holding part 140, and the ground electrode 117x and the sensor electrode 117y of the sensor 117a face each other via the holding part 140.

The electrodes in which large change in capacitance occur, when an object of detection is brought into contact therewith, are the sensor electrodes 117y. Thus, if the above-explained construction in which sensor electrodes are arranged in different positions in the longitudinal direction of the holding part 140 is adopted, the probability that both the sensors 117a and 117b erroneously detect, at the same time, a foreign substance such as the cleaning swab 50 as the stick-type base material 150 becomes very low; this is because, for example, even in the case that L2 is shorter than L1 in (b) in Fig. 8 and a state that a foreign substance (the cleaning swab 50 in the present example) touches both sensors 117a and 117b has happened, if the above state is that the foreign substance touches the sensor electrode 117y in one sensor and does not touch the sensor electrode 117y and touches the ground electrode 117x in the other sensor, change in capacitance of the other sensor is small.

That is, by arranging the sensor electrode 117y of the sensor 117a and the ground electrode 117x of the sensor 117b in positions that substantially overlap with each other in the longitudinal direction of the holding part 140 (i.e., when they are viewed in the direction from the opening 143 to the bottom part 143), and by arranging the ground electrode 117x of the sensor 117a and the sensor electrode 117y of the sensor 117b in positions that substantially overlap with each other in the longitudinal direction of the holding part 140 (i.e., when they are viewed in the direction from the opening 143 to the bottom part 143), it becomes possible to precisely detect whether or not a detected object is that expected.

In the case of the aerosol generation device 100 illustrated in each of Figs. 8, 9, and 10, the controller 116 judges that the stick-type base material 150 has been inserted into the holding part 140, if a moving average value of plural capacitance-sensor values detected by using each of the sensors 117a and 117b or difference between two moving average values has exceeded a threshold value that was determined in relation to the moving average value or the difference. Further, the threshold value relating to the capacitance-sensor value detected by using the sensor 117a and that relating to the capacitance-sensor value detected by using the sensor 117b may be the same with each other or different from each other.

Although the construction that the aerosol generation device 100 comprises two sensors are illustrated in each of Figs. 8, 9, and 10, the aerosol generation device 100 may comprise three or more sensors. Further, it may be constructed in such a manner that the controller 116 makes a judgment as to whether a moving average value of plural capacitance-sensor values detected by using each of the sensors or difference between two moving average values has exceeded a threshold value that was determined in relation to the moving average value or the difference. By adopting the above construction, accuracy of detection of the stick-type base material 150 can be further improved.

Further, it may be constructed in such a manner that, in the case that change in capacitance in only one of the sensors 117a and 117b is detected by the controller 116, the notifier 113 outputs a notification that encourages a user to perform cleaning of the stick-type base material 150, since it is highly likely that a foreign substance which is not the stick-type base material 150 has been detected.

Further, at least one of two or more the sensors may be a sensor other than a capacitance sensor, for example, an optical sensor such as an infrared proximity sensor or the like, a pressure sensor using a piezoelectric element, or the like.

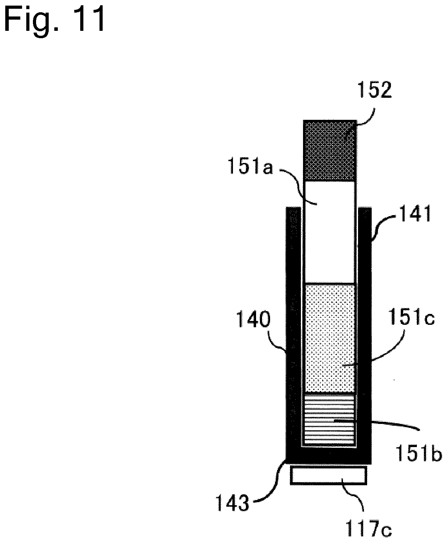

Fig. 11 is a figure which shows an example of a construction in which plane electrodes included in a sensor 117c are arranged to face the bottom part 143 of the holding part 140 in the aerosol generation device 100. The sensor 117c is a component of the detector 117, and is used for detecting capacitance of the capacitor Cx. The sensor 117c may further be connected to the sampling capacitor Cs to constitute the detector 117. In this regard, to constitute the capacitor Cx, the plane electrodes included in the sensor 117c include a ground electrode that is positioned on a surface opposite to the surface facing the holding part 140. Further, Fig. 11 shows the state that the stick-type base material 150 is being inserted into the holding part 140. In the case that the plane electrodes are arranged as explained above, the detector 117 can detect, as the capacitance of the capacitor Cx, the capacitance formed by a combination of the plane electrodes and the stick-type base material 150, when the stick-type base material 150 is being inserted into the holding part 140.

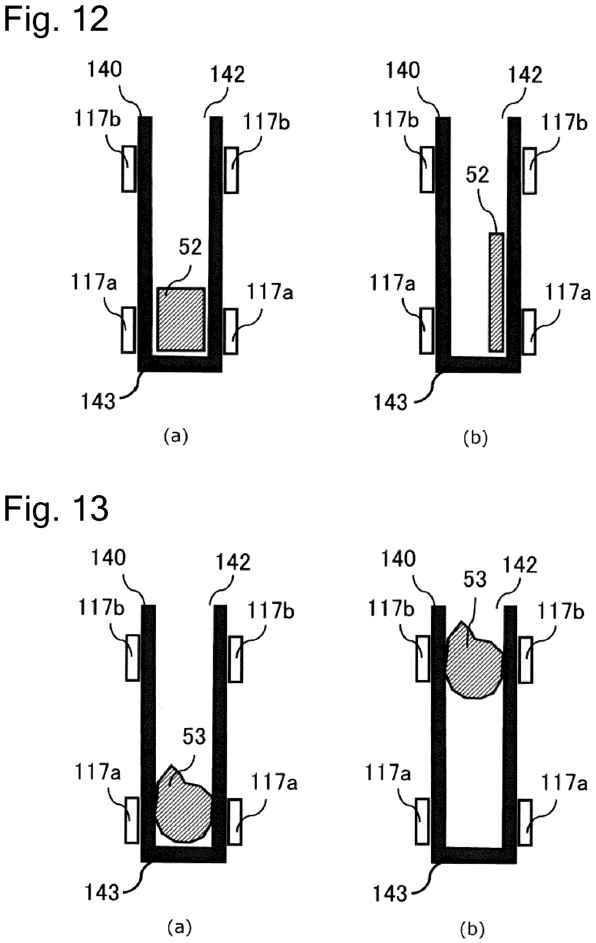

Each of Fig. 12 and Fig. 13 is a figure which shows an example of a case that a foreign substance has entered the holding part 140. Fig. 12 illustrates a case that a liquid foreign substance such as water, glycerin, or the like has entered the holding part 140. Fig. 13 illustrates a case that a foreign substance (especially, a solid foreign substance) different from that explained above has entered the holding part 140.

In the case that a liquid foreign substance has entered the holding part 140, it can be assumed that there may be a case that a foreign substance 52 adherers the holding part 140 in such a manner that it covers the whole bottom part 143 as shown in (a) in Fig. 12, and a case that the foreign substance 52 adherers to the side surface in the inside of the holding part 140 as shown in (b) in Fig. 12.

On the other hand, in the case that a solid foreign substance has entered the holding part 140, it can be assumed that there may be a case that a foreign substance 53 adherers the holding part 140 in such a manner that it covers the whole bottom part 143 as shown in (a) in Fig. 13, and a case that the foreign substance 53 adherers to a part close to the opening 142 of the holding part 140 as shown in (b) in Fig. 13.

When it is assumed that any of the above cases of entering of foreign substances may happen, it is preferable to make the sensor 117a comprise a capacitance sensor and make the sensor 117b comprise an optical sensor. It appears that, when compared with the case that a foreign substance stays on a part close to the opening 142, a foreign substance tends to stay on the bottom part 143 of the holding part 140, and, accordingly, the bottom part 143 tends to become unclean due to tobacco leaves or the like; so that it is more preferable if a sensor to be installed in a position close to the bottom part 143 is a capacitance sensor.

Further, there may be a case that additives such as glycerin and so on in the stick-type base material 150 is vaporized by applying heat. The capacitance changes as the quantity of additives changes; thus, in the case that additives such as glycerin and so on have been vaporized by applying heat, there may be a case that, even if insertion of the stick-type base material 150 has been completed, the count value does not reach the predetermined threshold value and insertion of the stick-type base material 150 is not detected accordingly. Thus, for example, it may be constructed in such a manner that a capacitance sensor is adopted as the sensor 117a and an optical sensor is adopted as the sensor 117b; and sensor values of the two sensors 117a and 117b are detected at the time when the stick-type base material 150 is inserted for starting heating or the like, and the sensor 117a in a position close to the bottom part 143 is not used and the sensor 117b in a position close to the opening 142 only is used after starting heating or after a predetermined length of time has elapsed since a start of heating.

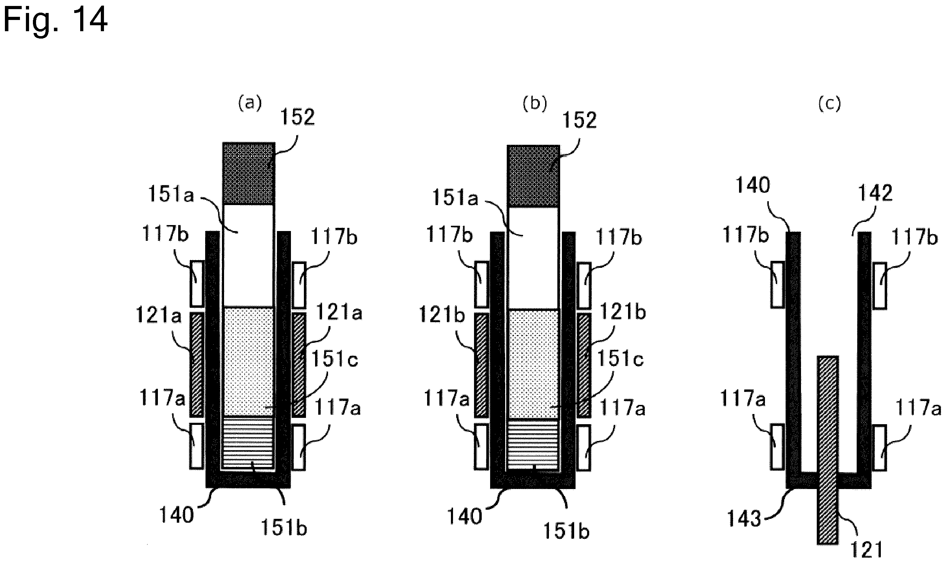

Fig. 14 is a figure which shows examples of arrangement of detectors 117 and heaters 121 in aerosol generation devices 100 according to embodiments of the present invention. The above-explained construction comprising plural sensors (detectors 117) can be applied to the aerosol generation device 100 regardless of whether it adopts an externally heating system or an internally heating system. (a) in Fig. 14 shows a construction example of an externally heating system using a heater. (b) in Fig. 14 shows a construction example of an electromagnetic induction heating system using a coil. (c) in Fig. 14 shows a construction example of an internally heating system using a heater. As shown in each of (a) and (b) in Fig. 14, in the case of the externally heating system, it may be possible to adopt a construction in which a heater 121a or a coil 121b (the heater 121) is installed in a position between the sensor 117a and the sensor 117b. According to the above construction, it becomes possible to arrange the heater 121a or the coil 121b in a positon corresponding to that of the material part 151c in the stick-type base material 150, and arrange the sensor 117a and the sensor 117b, that are used for detecting capacitance, in positions corresponding to the paper tube 151a and the paper filter 151b which are positioned above and below the material part 151c, respectively. By adopting the above construction, it becomes possible to adjust the quantity of glycerin or PG (propylene glycol) to that appropriate for detection of capacitance, and add it to the paper tube 151a and/or the paper filter 151b rather than the material part 151c. Glycerin or PG (propylene glycol) is also used as an aerosol source; however, by adopting the above construction, heating is effectively performed to deliver the aerosol included in the material part 151c mainly to a user, and glycerin or PG (propylene glycol) added to the paper tube 151a and/or the paper filter 151b can be made less likely to be vaporized compared with that in the material part 151c; accordingly, even in the case that heating is being performed, detection of capacitance by the sensors 117a and 117b can be made easier.

In (c) in Fig. 14, the heater 121c (the heater 121) is constructed to have a blade shape, and arranged in such a manner that it protrudes from the bottom part 143 to the inner space 141 of the holding part 140. In the case that the hater 121 has a construction such as that explained above, the sensor 117a and the sensor 117b may be arranged in positions separate from each other; specifically, one sensor may be arranged in a position close to the bottom part 143 and the other sensor may be arranged in a position close to the opening 142 in the holding part 140.

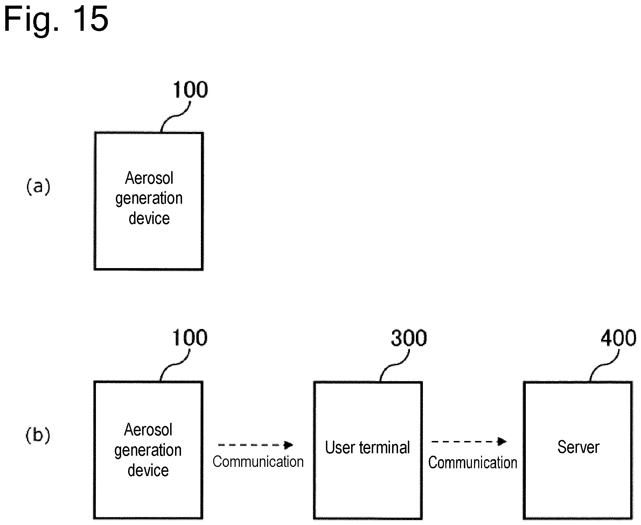

It may be constructed in such a manner that a notification that encourages a user to perform cleaning of the stick-type base material 150 is outputted, in the case that a foreign substance in the holding part 140 is detected by the sensor 117 as explained above. Fig. 15 is a figure which shows a system construction example in the case that a notification is presented to a user. (a) in Fig. 15 shows a case that the aerosol generation device 100 itself presents a notification to a user. In the above case, a notification can be outputted to a user by using a user interface (the notifier 113) included in the aerosol generation device 100. More specifically, a notification that encourages a user to perform cleaning of the stick-type base material 150, that is in the form of sound, light, vibration, characters, an image, or the like, can be presented by using a sound outputting device such as a speaker or the like, a light emitting element such as an LED (light emitting diode) or the like, a vibrator function realized by using a vibrating element, a display device such as a liquid crystal panel or the like.

On the other hand, (b) in Fig. 15 shows a case that the aerosol generation device 100 presents, via other devices 300 and 400, a notification to a user. For example, by using, as a trigger, an event that the controller 116 has made a judgment indicating entering of a foreign substance, it may be possible to present data of a message, an image, or the like that encourages a user to perform cleaning, by transmitting the data to the user terminal 300 such as a smartphone, a tablet, or the like possessed by the user by using a communication function (the communicator 115) such as Bluetooth (a registered trademark) or the like installed in the aerosol generation device 100. Further, it may be constructed in such a manner that, by using, as a trigger, an event that the controller 116 has made a judgment indicating entering of a foreign substance, entering-of- foreign-substance information including result of judgment in the controller 116 with respect to a foreign substance and so on is transmitted to the user terminal 300 such as a smartphone, a tablet, or the like possessed by a user by using a communication function (the communicator 115) such as Bluetooth (a registered trademark) or the like installed in the aerosol generation device 100, and the entering-of-foreign-substance information is further transmitted from the user terminal 300 to the server 400. By adopting the above construction, it becomes possible to accumulate entering-of-foreign-substance information and study the frequency of occurrence of entering of a foreign substance and so on in the server 400. For example, if it becomes possible to detect dirt, it becomes possible to study, based on data of the number of times of smoking and the entering-of-foreign-substance information that are appropriately transmitted from the aerosol generation device 100 to the server 400, the frequency of cleaning in relation to the number of times of smoking.

### (Flow of Process in Aerosol Generation Device 100)

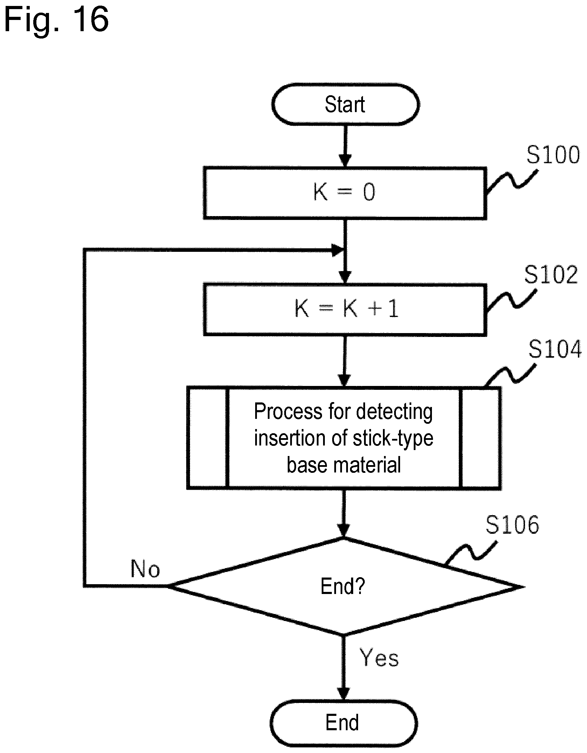

Each of Fig. 16 and Fig. 17 shows an example of a flow of a process performed in the aerosol generation device 100. Fig. 16 is a figure which shows an example of a main process flow. For example, during the time when the aerosol generation device 100 is active (during the time when the electric power supply 111 has electric power that is sufficient for the aerosol generation device 100 to function), the process illustrated in Fig. 16 may start at timing when an explicit instruction is inputted as a result of manipulation performed by a user, or the like.

In Fig. 16, first, the controller 116 initializes a variable K (sets it to K="0") (step S100). The variable K is a variable representing the count of the cycles for calculating moving averages, thus, representing the cycle number of the present cycle. The variable K is used in the process flow in Fig. 17.

Next, the controller increments the variable K by 1 (step S102). The controller 116 and the detector 117 perform the process for detecting insertion of the stick-type base material 150 (step S104). The insertion detecting process in step S104 may be performed in accordance with the process flow illustrated in Fig. 17. The process is repeated until a judgment indicating an end is made (step S106). In this regard, the condition that may be regarded as an end is, for example, turning off of an electric power supply (a dead battery state), inputting of an explicit instruction by user manipulation, or the like, and an ending process may be performed at timing when the above condition is met.

Fig. 17 is a figure which shows an example of a process flow of the process for detecting insertion of the stick-type base material 150 in step S104 in Fig. 16.

First, by using, as a trigger, an event such as turning on of the electric power supply in the aerosol generation device 100, disconnecting of the charging terminal PG1 from the charger 200, or the like, the controller 116 performs initialization of ent that is a count value for counting the number of times that operation for transferring electric charge from the capacitor Cx to the sampling capacitor Cs is performed (sets it to cnt="0") (step S202). The controller 116 makes, after a predetermined length of time has elapsed, the capacitor Cx in the detector 117 (the capacitance sensor) transfer electric charge therein to the sampling capacitor Cs (step S204). The controller 116 increments the value of ent by 1 (step S206). The controller 116 performs judgment as to whether the terminal voltage of the sampling capacitor Cs is equal to or greater than "Vih" (step S208). If the terminal voltage of the sampling capacitor Cs is less than "Vih" (step S208: No), the controller 116 repeats the process to transfer electric charge in the capacitor Cx to the sampling capacitor Cs at predetermined time intervals (steps S204 and S206). If the terminal voltage of the sampling capacitor Cs has become that equal to or greater than "Vih" (step S208: Yes), the value of ent in a single sampling process can be obtained. The controller 116 stores the value of ent in a memory or the like (the memory 114) (step S210).

The detector 117 and the controller 116 repeat processes in steps S202-S210 at predetermined sampling periods, until count values for X times are obtained, wherein X is a predetermined number (step S212: No). In this regard, "X" is the number of times of sampling processes performed for obtaining a moving average. For example, in the case of the example in Fig. 7, a moving average of count values obtained by performing three sampling processes is calculated, thus, X=3 in the case.

After obtaining count values for X times (step S212: Yes), the controller 116 calculates a moving average of the count values for X times (step S214). Thereafter, the controller 116 calculates difference Y between the moving average of the count values for X times and the moving average obtained at the time of the (K-1)th time (the last moving average that was calculated when the process in Fig. 17 was performed last time) (step S216). The controller 116 performs judgment as to whether the calculated difference Y is greater than a threshold value Th that has been set in relation to difference (step S218). If the difference Y is greater than the threshold value Th (step S218: Yes), the controller 116 makes a judgment indicating a state that the stick-type base material 150 is not being inserted into, or has been pulled out of, the holding part 140 (step S220). If the difference Y is equal to or less than the threshold value Th (step S218: No), the controller 116 makes a judgment indicating a state that the stick-type base material 150 is being inserted into the holding part 140 (step S222).

In this regard, for example, with respect to moving averages, each being a moving average for three times (X=3), they may be obtained by calculating a moving average of values obtained in (n)th time, (n+1)th time, and (n+2)th time, calculating a moving average of values obtained in (n+3)th time, (n+4)th time, and (n+5)th time, and so on, or they may be obtained by calculating a moving average of values obtained in (n)th time, (n+1)th time, and (n+2)th time, calculating a moving average of values obtained in (n+1)th time, (n+2)th time, and (n+3)th time, and so on. In the above case, after the values of ent for X times are obtained first, the controller 116 stores the values of ent in a memory or the like (the memory 114) in step S210, and, thereafter, the process proceeds to step S214.

It should be reminded that the process flow shown in each of Fig. 16 and Fig. 17 is a process flow that has been constructed on the assumption that the sampling process is repeated, for example, from the time when the electric power supply is turned on to the time when the electric power supply is turned off. In this regard, for example, it may be constructed in such a manner that the sampling process is performed during a predetermined length of time that is determined by using a timer.

### (Other embodiments)

The controller 116 may be constructed in such a manner that it changes the sampling period for detecting the value of the capacitance sensor in the detector 117, based on detection of completion of connection between the charging terminal and an electric power supplying source such as an external charger, an external electrical outlet, or the like. For example, the controller 116 may control the detector 117 to suspend detection of the value of the capacitance sensor during the time when electric power is being supplied from the outside of the aerosol generation device 100 to the electric power supply 111. Especially, in a system of a PCC, electric power that is sufficient for allowing several number of times of smoking is supplied from a charger to the electric power supply 111; however, since the charger itself is portable and the electric power stored in the charger is limited, it is desired to save the electric power as much as possible. Further, in general, during a charging state, a user is in the state that the user does not use the aerosol generation device 100 (i.e., the user does not perform smoking). Thus, the electric power may be saved by adopting a construction that the process relating to the detector 117 is not performed during charging.

During a predetermined length of time after stopping of supply of electric power from the outside, such as a separate charger, an electrical outlet, or the like, the controller 117 may change the sampling period for detecting the capacitance-sensor value to that shorter than a sampling period in the time after the elapse of the predetermined length of time. It is anticipated that a user starts use of the aerosol generation device 100 (i.e., starts smoking) right after the time when charging is completed or suspended. That is, it is highly likely that the stick-type base material 150 will be inserted for smoking. Thus, in anticipation of insertion of the stick-type base material 150, the sampling period for detecting the value of the capacitance sensor in the detector 117 may be made short during a predetermined length of time after stopping of charging, and the sampling period may be made long after the elapse of the above length of time. In this regard, the expression "detecting the value of the capacitance sensor" corresponds to a case that a value relating to parasitic capacitance is to be detected and a case that a value relating to a sampling capacitor is to be detected.

Further, during the time when the state that electrical and/or physical connection to a separate charger is being detected by the controller 116, the controller 116 may control the detector 117 to refrain from performing detection of the capacitance-sensor value. The "electrical connection" includes connection using a charging terminal, connection using a different method such as noncontact connection and so on.

During a predetermined length of time after disconnecting the electrical and/or physical connection to the charger, the controller 116 may control the detector 117 to perform detection of the capacitance-sensor value at a sampling period shorter than a sampling period during the time after the elapse of the above length of time. It is anticipated that a user starts use of the aerosol generation device 100 (i.e., starts smoking) after disconnecting of connection to the charger, that is, after completing or suspending of charging. Thus, in anticipation of insertion of the stick-type base material 150, the sampling period for detecting the value of the capacitance sensor of the detector 117 may be made short during a predetermined length of time after disconnecting of connection to the charger, and the sampling period may be made long after the elapse of the above length of time.

Further, it may be constructed in such a manner that the holding part 140 comprises a lid 130 which can be opened/closed, and the controller 116 can detect an open or close state of the lid 130. For example, the lid 130 moves, by using a hinge or a slider, between a position for covering the opening formed for inserting the stick-type base material 150 (a close state) and a position for opening the opening (a open state). The stick-type base material 150 is not inserted during the time when the lid 130 is being closed; thus, in view of electric power saving, the controller 116 may perform control to refrain from performing detection of the capacitance-sensor value. In this regard, in the case that the holding part 140 does not comprise the lid 130, it is always anticipated that the stick-type base material 150 will be inserted; thus, it is preferable that the process for detecting insertion of the stick-type base material 150 be performed always. In the above case, it is effective to change sampling, based on the state of connection of the charging terminal explained above, or the like.

In the above description, embodiments of the present disclosure have been explained together with their modification examples and application modes; and, in this regard, it should be understood that they are mere examples, and they are not those limiting the scope of the present disclosure. It should be understood that change, addition, modification, and so on with respect to the embodiments can be performed appropriately, without departing from the gist and the scope of the present disclosure. The scope of the present disclosure should not be limited by any of the above-explained embodiments, and should be defined by the claims and equivalents thereof only.

Further, the constructions such as those shown below are constructions within the scope of the technique of the present invention.

(1A) An aerosol generation device for generating aerosol, comprising:
a holding part for holding an aerosol forming base body comprising an aerosol source;
a heater for heating the aerosol source;
a detector for performing detection of plural values of a capacitance sensor; and
a controller which calculates a moving average value of the detected plural values of the capacitance sensor, and makes, by using the calculated moving average value and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

(2A) The aerosol generation device as recited in item (1A), wherein
the threshold value is a threshold value relating to difference between the two moving average values, and
the controller makes, by comparing the difference between the two moving average values with the threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

(3A) The aerosol generation device as recited in item (1A) or (2A), wherein the controller is able to perform operation in a first mode and a second mode, wherein consumption of electric power in the first mode is different from that in the second mode; the consumption of electric power in the second mode is smaller than that in the first mode; and, during the second mode, the controller cancels the second mode after the time when a predetermined length of time has elapsed, and makes the detector perform the detection.

(4A) The aerosol generation device as recited in any one of items (1A)-(3A), wherein the detector uses a sampling capacitor.

(5A) The aerosol generation device as recited in any one of items (1A)-(4A), comprising
an electric power supply which is able to store electric power, and a charging terminal which is to be electrically connected to the electric power supply; wherein
the controller changes, based on an event that completion of connection between the charging terminal and an external electric power supplying source is detected, a sampling period used for detecting values of the capacitance sensor in the detector.

(6A) The aerosol generation device as recited in item (5A), wherein the detector does not perform detection of the values of the capacitance sensor, during the time when electric power is being supplied to the electric power supply from the outside of the aerosol generation device.

(7A) The aerosol generation device as recited in item (5A) or (6A), wherein, during a predetermined length of time after a stop of supply of electric power from the outside, the detector makes the sampling period used for detecting the values of the capacitance sensor shorter than a sampling period during the time after the elapse of the predetermined length of time.

(8A) The aerosol generation device as recited in any one of items (1A)-(7A), wherein
the aerosol generation device can be electrically connected to a separate charger which supplies electric power to the aerosol generation device, and
the detector does not perform detection of the values of the capacitance sensor, during the time when connection to the charger is being made.

(9A) The aerosol generation device as recited in item (8A), wherein, during a predetermined length of time after disconnection of connection to the charger, the detector performs detection of the values of the capacitance sensor at a sampling period shorter than a sampling period during the time after the elapse of the predetermined length of time.

(10A) The aerosol generation device as recited in any one of items (1A)-(9A), wherein
the holding part comprises a lid which can be opened/closed, and
the detector does not perform detection of the values of the capacitance sensor, during the time when the lid is being closed.

(11A) The aerosol generation device as recited in any one of items (1A)-(10A), wherein the number of the detectors is at least two.

(12A) The aerosol generation device as recited in item (11A), wherein the threshold values used in the at least two detectors, respectively, are different from one another.

(13A) The aerosol generation device as recited in item (11A) or (12A), wherein
the holding part has an insertion opening used for inserting the aerosol forming base body, and a slender space shape for holding the aerosol forming base body, and,
in the at least two detectors, a first detector is arranged in a position on an insertion-opening side in a longitudinal direction of the slender space shape in the holding part, and a second detector is arranged in a position on a side opposite to the insertion-opening side, where the first detector is positioned, in the longitudinal direction.

(14A) The aerosol generation device as recited in item (13A), wherein
the first detector comprises a first electrode and a second electrode which face with each other in a direction perpendicular to the longitudinal direction,
the second detector comprises a third electrode and a fourth electrode which face with each other in a direction perpendicular to the longitudinal direction,
the first electrode and the third electrode are connected to the ground,
the first electrode and the fourth electrode are arranged in positions that overlap with each other when they are viewed in the longitudinal direction, and the second electrode and the third electrode are arranged in positions that overlap with each other when they are viewed in the longitudinal direction.

(15A) The aerosol generation device as recited in any one of items (11A)-(14A), wherein the heater is arranged in a position between the at least two detectors.

(16A) The aerosol generation device as recited in any one of items (11A), (12A), (13A), and (15A), wherein at least one of the at least two detectors is a sensor other than a capacitance sensor.

(17A) The aerosol generation device as recited in any one of items (11A)-(16A), wherein the controller makes, based on result of detection that the aerosol forming base body has been inserted obtained by each of the at least two detectors, a judgment indicating existence of a substance other than the aerosol forming base body in the holding part.

(18A) The aerosol generation device as recited in item (17A) further comprising a notifier which outputs a notification for informing a user of existence of a substance other than the aerosol forming base body in the holding part, or a notification for encouraging a user to perform cleaning of the holding part, if the controller has made a judgment indicating existence of a substance other than the aerosol forming base body in the holding part.

(19A) A method performed by an aerosol generation device, which comprises a holding part for holding an aerosol forming base body comprising an aerosol source and a heater for heating the aerosol source, comprising steps for:
detecting plural values of a capacitance sensor; and
calculating a moving average value of the detected plural values of the capacitance sensor, and, by using the calculated moving average value and a predetermined threshold value, making at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

(20A) A program which makes an aerosol generation device perform the method recited in above item (19A).

(1B) An aerosol generation device for generating aerosol, comprising:
a holding part for holding an aerosol forming base body comprising an aerosol source;
a heater for heating the aerosol source;
a detector for performing detection of a value of a capacitance sensor; and
a controller which makes, by using the detected value of the capacitance sensor and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed; wherein
the controller changes, based on a state of heating control relating to the heater, a sampling period used for detecting the value of the capacitance sensor.

(2B) The aerosol generation device as recited in item (1B), wherein the controller changes the sampling period used for detecting the value of the capacitance sensor, depending on whether or not the heater is heating the aerosol forming base body.

(3B) The aerosol generation device as recited in item (1B) or (2B), wherein the detector uses a sampling capacitor.

(4B) The aerosol generation device as recited in any one of items (1B)-(3B), wherein the sampling period during the time when the heater is heating the aerosol forming base body is longer than the sampling period during the time when heating is being stopped.

(5B) The aerosol generation device as recited in any one of items (1B)-(4B), wherein the sampling period during the time when the heater is heating the aerosol forming base body is shorter than the sampling period during the time when heating is being stopped.

(6B) The aerosol generation device as recited in any one of items (1B)-(5B), wherein the controller calculates a moving average value of the detected plural values of the capacitance sensor, and makes, by using the calculated moving average value and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

(7B) The aerosol generation device as recited in item (6B), wherein
the threshold value is a threshold value relating to difference between the two moving average values, and
the controller makes, by comparing the difference between the two moving average values with the threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

(8B) The aerosol generation device as recited in any one of items (1B)-(7B), wherein the controller is able to perform operation in a first mode and a second mode in which consumption of electric power is different from that in the first mode; the consumption of electric power in the second mode is smaller than that in the first mode; and, after heating of the aerosol forming base body by the heater is stopped, the mode of the aerosol generation device is changed to the second mode if a predetermined condition is satisfied, and the state in the second mode is canceled at predetermined time intervals to allow the detector to perform detection.

(9B) The aerosol generation device as recited in any one of items (1B)-(8B), wherein the number of the detectors is at least two.

(10B) The aerosol generation device as recited in item (9B), wherein the controller makes at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using each of the values of the capacitance sensors, that are detected in the at least two detectors, and the threshold value.

(11B) A method performed by an aerosol generation device, which comprises a holding part for holding an aerosol forming base body comprising an aerosol source and a heater for heating the aerosol source, comprising steps for:
performing detection of a value of a capacitance sensor; and
making at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using the detected value of the capacitance sensor and a predetermined threshold value; wherein
the step for making the judgment changes, based on a state of heating control relating to the heater, a sampling period used for detecting the value of the capacitance sensor.

(12B) A program which makes an aerosol generation device perform the method recited in above item (11B).

### REFERENCE SIGNS LIST

100 ... Aerosol generation device
111 ... Electric power supply
112 ... Sensor
113 ... Notifier
114 ... Memory
115 ... Communicator
116 ... Controller
117 ... Detector
121 ... Heater
130 ... Lid
140 ... Holding part
141 ... Inner space
142 ... Opening
143 ... Bottom part
144 ... Heat insulator
150 ... Stick-type base material
151 ... Base-material part
152 ... Suction opening (Mouthpiece)
200 ... Charger
300 ... User terminal
400 ... Server
50 ... Cleaning swab
52, 53 ... Foreign substance

## Claims

1. An aerosol generation device for generating aerosol, comprising:
a holding part for holding an aerosol forming base body comprising an aerosol source;
a heater for heating the aerosol source;
a detector for performing detection of a value of a capacitance sensor; and
a controller which makes, by using the detected value of the capacitance sensor and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed; wherein
the controller changes, based on a state of heating control relating to the heater, a sampling period used for detecting the value of the capacitance sensor.

2. The aerosol generation device as recited in Claim 1, wherein the controller changes the sampling period used for detecting the value of the capacitance sensor, depending on whether or not the heater is heating the aerosol forming base body.

3. The aerosol generation device as recited in Claim 1 or 2, wherein the detector uses a sampling capacitor.

4. The aerosol generation device as recited in any one of Claims 1-3, wherein the sampling period during the time when the heater is heating the aerosol forming base body is longer than the sampling period during the time when heating is being stopped.

5. The aerosol generation device as recited in any one of Claims 1-4, wherein the sampling period during the time when the heater is heating the aerosol forming base body is shorter than the sampling period during the time when heating is being stopped.

6. The aerosol generation device as recited in any one of Claims 1-5, wherein the controller calculates a moving average value of the detected plural values of the capacitance sensor, and makes, by using the calculated moving average value and a predetermined threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

7. The aerosol generation device as recited in Claim 6, wherein
the threshold value is a threshold value relating to difference between the two moving average values, and
the controller makes, by comparing the difference between the two moving average values with the threshold value, at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed.

8. The aerosol generation device as recited in any one of Claims 1-7, wherein the controller is able to perform operation in a first mode and a second mode in which consumption of electric power is different from that in the first mode; the consumption of electric power in the second mode is smaller than that in the first mode; and, after heating of the aerosol forming base body by the heater is stopped, the mode of the aerosol generation device is changed to the second mode if a predetermined condition is satisfied, and the state in the second mode is canceled at predetermined time intervals to allow the detector to perform detection.

9. The aerosol generation device as recited in any one of Claims 1-8, wherein the number of the detectors is at least two.

10. The aerosol generation device as recited in Claim 9, wherein the controller makes at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using each of the values of the capacitance sensors, that are detected in the at least two detectors, and the threshold value.

11. A method performed by an aerosol generation device, which comprises a holding part for holding an aerosol forming base body comprising an aerosol source and a heater for heating the aerosol source, comprising steps for:
performing detection of a value of a capacitance sensor; and
making at least one of a judgment as to whether inserting of the aerosol forming base body into the holding part has been completed, a judgment as to whether or not the aerosol forming base body has been inserted into the holding part, and a judgment as to whether extracting of the aerosol forming base body from the holding part has been completed, by using the detected value of the capacitance sensor and a predetermined threshold value; wherein
the step for making the judgment changes, based on a state of heating control relating to the heater, a sampling period used for detecting the value of the capacitance sensor.

12. A program which makes an aerosol generation device perform the method recited in Claim 11.
